# EUROPEAN PATENT SPECIFICATION

(11) **EP 3 019 132 B1**
(45) Date of publication and mention of the grant of the patent: **13.09.2023**
(21) Application number: 14739661.8
(22) Date of filing: 03.07.2014
(51) Int. Cl.: A61F 13/42

(54) **SYSTEM FOR MEASURING THE QUANTITY OF URINE AND DETECTING THE PRESENCE OF FAECES IN A NAPPY**
SYSTEM ZUR MESSUNG EINER URINMENGE UND ZUR FESTSTELLUNG DES VORHANDENSEINS VON FÄKALIEN IN EINER WINDEL
SYSTÈME POUR MESURER LA QUANTITÉ D'URINE ET DÉTECTER LA PRÉSENCE DE SELLES DANS UNE COUCHE

(30) Priority: 12.07.2013 DK 201300425
(43) Date of publication of application: 18.05.2016
(73) Proprietor: Wear&Care ApS, 2670 Greve (DK)
(72) Inventor: ETTRUP HANSEN, Martin, 5300 Kerteminde (DK)
(74) Representative: Chas. Hude A/S
(86) International application number: PCT/DK2014/050199
(87) International publication number: WO 2015/003712

(56) References cited:
- WO-A1-2008/026092
- WO-A1-2013/016765
- WO-A1-2013/091728
- US-A- 4 653 491
- US-A1- 2004 207 530
- US-A1- 2008 278 337
- US-A1- 2010 241 094

## Description

The present invention relates to a system for measuring the quantity of urine in an absorbing material and, in some embodiments, detecting the presence of faeces, for example in a nappy, wherein one or more capacitive sensors are used to determine the state of fullness of liquid in the absorbing material and for detecting the presence of faeces.

### Background of the invention.

Alarms used in connection with measuring the presence of urine in nappies, underwear and sheets, etc. are known in the art in the form of various enuresis and incontinence products. Urine sensors in nappies are known in the prior art, but there are none for measuring faeces.

The conventional products are characterised by their emission of an alarm as soon as a liquid is present on the sensor, a so-called instant alarm. The products typically function by making a resistive measurement across a sensor in which no current is passing between the electrodes in a normal situation (when the nappy is dry). In an alarm situation, an electrical connection is created between the electrodes via the liquid between the electrodes, following which a given current will pass from one electrode to the other. The liquid, in these cases urine, works as a simple contact between two or more electrodes.

Examples of such systems are described in US 7,700,821 B2 and US 7,667,608 B2, and the object of such systems is precisely to produce an alarm as quickly as possible, as urine on the sensor is an undesirable situation for children who are bed-wetters or for incontinence patients. The conventional resistive instant alarms are found in countless variants and are, as noted above, included in nappies, panty liners, underwear, sheets, underlays and beds etc., all being characterised by the above technology and function.

Systems also exist for detecting quantities of liquid in, for example, nappies, which systems are characterised in being embedded in the liquid-absorbing material or in measuring through the liquid-absorbing material in the nappy. Examples of such systems are described in US 2008/0278337 A1 and WO 2013/013197 A1, the object of which systems is to signal a degree of wetness in the liquid-absorbing material in a nappy.

WO 2013/016765 A1 discloses a sensor device for sensing wetness in an absorbent article worn by a subject includes one or more sensing elements; and a coupling for communicating sensor signals between the one or more sensing elements and a receiver. A change in environmental parameter causes a change in electrical behaviour of at least one of the sensing elements, which behaviour can be analysed to determine occurrence of a wetness event in the absorbent article. The changes in electrical behaviour are communicated in the sensor signals to the receiver.

US 2010/0241094 A1 discloses an absorbent article, which has an outer cover, an inner absorbent material and a sensing system. The sensing system has a measuring device, a monitoring area and a warning device. The monitoring area has a plurality of conductors, which are in electrical engagement with the measuring device. The conductors of the monitoring area are spaced apart to sense if an insult has occurred and to sense the size of the insult. A warning device is electrically connected to the measuring device. The warning device provides information to the caregiver regarding the occurrence and size of the liquid or solid insult.

WO 2013/091728 A1 discloses a method for detecting a liquid discharge event in an absorbent article, wherein said absorbent article comprises a sensor adapted to generate an electrical output signal representative of a degree of wetness of said absorbent article, wherein said electrical output signal is received by a processing unit. The invention relates also to a system for detecting liquid discharge event in an absorbent article, said system comprising an absorbent article comprising a sensor being arranged to generate an output signal representative of an electrical property, and a processing unit adapted to process said output signal generated by the sensor of the absorbent article.

US 2008/0278337 A1 discloses a urine detection system for detecting degree of wetness of a diaper, comprising a plane printing electrode, a sensor, and a display unit. The plane printing electrode comprises a first electrode area and a second electrode area. The sensor comprises a first sensor electrode, a second sensor electrode and a processor. The first sensor electrode and the first electrode area forms a first capacitor, and the second sensor electrode and the second electrode area forms a second capacitor. The processor detects capacitance of the first and second capacitors, and determines a signal representing degree of wetness of the diaper. The display unit receives the signal and displays the degree of wetness corresponding to the signal.

US 2004/0207530 A1 discloses an elimination-absorber monitoring system addresses diaper-monitoring problems with a unique, low cost, multi-layer disposable sensor structure that absorbs small volumes of urine, yet allows most urine volume to flow unimpeded through it, and into the diaper below. When connected with a reusable, miniature monitor/indicator unit, the sensor presents a clear and on-going change of measurement condition upon experiencing a rapid influx into the diaper of a significant volume of urine, and/or upon a significant reduction in the available absorbency of the diaper's top surface.

US 4,653,491 A discloses a water content sensing and informing system for a disposable diaper consisting of water permeable inner sheet, water absorber, and water impermeable outer sheet, comprises at least one pair of metal layers, one surface of one layer being covered with an electric insulating layer, formed between the water impermeable outer sheet and the water absorber, a detector for detecting water content absorbed in the diaper, and an informing mechanism for informing the detected result.

WO 2008/026092 A1 discloses a sensing system for use in conjunction with a home automation network controlling an environmental variable in a home, the system including a transmitter adapted to be used with an absorbent article having a sensor, wherein the transmitter is adapted to communicate with the sensor and directly with the home automation network.

### Brief description of the invention

It is an object of the present invention to provide a more advanced alarm system, which does not produce an either/or alarm, but uses a more differentiated measuring method with the possibility of self-selected alarm levels, which can be adapted to the institution or wearer. In this way, the system cannot only enhance the possibilities for improving the conditions for the nappy wearer, but also the working environment and the possibilities of preventing inappropriate situations.

Such a system is provided in the present invention, as defined by claim 1, which relates to system for measuring and recording a quantity of liquid in an absorbing material, which system comprises a sensor comprising a first pair of electrodes for measuring the quantity of liquid, which first pair of electrodes is arranged to be placed around the absorbing material, and a data logger, which is arranged to apply an alternating voltage across the first pair of electrodes and, concurrently herewith, to measure the capacitance between the two electrodes of the first pair of electrodes and to calculate, based on the measured capacitance, an estimate of the quantity of liquid in the absorbing material, characterised in that the sensor comprising the one pair of electrodes is arranged to be placed so that it extends along two opposite surfaces of the absorbing material. Further embodiments of the invention are defined by claims 2-14.

In an embodiment of the invention, the two opposite surfaces of the absorbing article are two surfaces of a nappy facing towards the wearer and away from the wearer, respectively.

Such a system is advantageous because it is able to continuously measure and pass on information on the quantity of liquid in a given absorbing item, such as a nappy.

Greater awareness of when the nappy needs changing can thus be obtained.

To ensure that the system is intact and that the capacitance measurements are correct, it will be advantageous, concurrently with the capacitance measurements, to check the so-called "dissipation factor", which is an expression of an undesirable resistive connection between the two electrodes between which the capacitance is measured. This can be done, for example, by measuring the ohmic resistance between the two electrodes.

In an embodiment of the invention, the sensor further comprises a second pair of electrodes, and the data logger is further arranged to be able to apply an alternating voltage across the second pair of electrodes and, concurrently herewith, to measure the capacitance between the two electrodes of the second pair of electrodes and to estimate, based on the measured capacitance, whether a substance, for example faeces, is present adjacent the two electrodes of the second pair of electrodes.

In an embodiment of the invention, the data logger further comprises a contact surface, which is arranged to be placed in electrical contact with a body of a person, and the data logger is further arranged to be able to apply an alternating voltage between the two electrodes of the second pair of electrodes on the one side and this contact surface one the other side and, concurrently herewith, measure the capacitance between the two electrodes of the second pair of electrodes on the one side and the contact surface on the other side and to estimate, based on the measured capacitance, whether a substance, for example faeces, is present between the two electrodes of the second pair of electrodes and the body.

In these embodiments, the invention can measure and pass on an alarm when faeces are present in the nappy, such that greater awareness can be obtained of when the nappy needs changing.

In an embodiment of the invention, a temperature-measuring instrument is arranged in connection with the contact surface of the data logger.

Such temperature-measuring instrument will, for example, be able to be used for checking that the surface is in fact in contact with the body as desired. It will also be able, in certain cases, to be used for measuring the person's body temperature.

In an embodiment of the invention, the data logger is provided with a gyroscope and/or an accelerometer.

The use of a gyroscope and/or an accelerometer makes it possible to determine the spatial orientation of the system (and of a person wearing the system), which makes it possible to adjust the measurement algorithm accordingly and obtain better utilisation of the filler capacity.

In an embodiment of the invention, the data logger is provided with a GPS tracking module for localising the data logger.

In an embodiment of the invention, the data logger is provided with a GSM modem for telecommunication with the data logger.

One segment to be helped by the invention is persons with impaired cognitive functions including Alzheimer's disease/dementia. In cases, where the consequence of this disease is a combination of incontinence and confusion, an embodiment with a GPS tracking system in the data logger can be a major advantage. In these cases, where the user has a requirement for both nappy and tracking, both can be combined in one unit. A combination of data logging and a GPS tracking system can also take advantage of the fact that a GPS tracking module and a GSM module can be switched off until the user is outside the local wireless network to which the data logger is normally connected, thus saving electricity.

In an embodiment of the invention, the alternating voltage applied has an amplitude of between 0.5 Vac and 15 Vac, preferably between 0.5 Vac and 10 Vac, most preferably between 1 Vac and 6 Vac.

In an embodiment of the invention, the alternating voltage applied has a frequency of between 1 kHz and 100 kHz, preferably between 5 kHz and 25 kHz, most preferably between 8 kHz and 12 kHz.

Amplitudes and frequencies within these intervals have proved to produce good measurement results and, furthermore, they mean that persons using the invention neither register nor are affected by the alternating voltage applied.

In an embodiment of the invention, the alternating voltage is applied for short periods at predefined intervals of between 5 seconds and 10 minutes, preferably between 5 seconds and 1 minute.

Application of the alternating voltage at said intervals has been found to produce good measurement results at the same time as electricity is saved.

In an embodiment of the invention, the data logger is further arranged to be able to transmit signals, which are representative of the calculated estimations of a quantity of liquid or of the presence of a substance, wirelessly to one or more external monitoring stations.

This allows for remote monitoring of the calculated estimations of the quantity of liquid and the presence of, for example, faeces. More specifically, this means that the invention can be arranged to emit a signal when a predefined optimal, uniform and objective criterion, for example for nappy changing, is met.

In an embodiment of the invention, the signals, which are representative of a quantity of liquid, represent an absolute quantity of liquid, such as for example 300 ml.

In an embodiment of the invention, the signals, which are representative of a quantity of liquid, represent a relative quantity of liquid, such as for example 20% of the absorbing material's total capacity.

Depending on the application of the invention, it may be most advantageous to define the system, such that either absolute or relative values are estimated and form the basis for determining whether a predefined criterion is met.

The fact that the sensor only extends on the surfaces of the absorbing material, for example a nappy, means that the sensor can be retrofitted without changing the original design of the product/material onto which it is fitted. The invention can be fitted on an arbitrary liquid-absorbing product/material, including, for example, all types of nappies, sanitary towels, panty liners etc.

### The figures

A few embodiments of the present invention are described in more detail below with references to the figures, of which
- Fig. 1: illustrates schematically a sensor for retrofitted use according to an embodiment of the invention as seen from the outside,
- Fig. 2: shows the sensor of Fig. 1 as seen from the rear,
- Fig. 3: shows the sensor of Fig. 1, wherein a part of the outer layer is removed to make internal layers of the sensor partly visible,
- Fig. 4: shows the sensor of Fig. 2, wherein a part of the rear side layer is removed to make internal layers of the sensor partly visible,
- Fig. 5: shows a cross-section of the sensor of Figs. 1 and 3 as indicated by the A-A line therein,
- Fig. 6: shows a cross-section of the sensor of Fig. 1 as indicated by the B-B line therein,
- Fig. 7: illustrates schematically a nappy as seen from the inside with the sensor of Figs. 1-6 fitted therein,
- Fig. 8: shows a cross-section of the nappy and sensor of Fig. 7 along the C-C line indicated therein with an indication of electrical fields,
- Fig. 9: shows a cross-section of the nappy and sensor of Fig. 7 along the D-D line indicated therein with an indication of electrical fields,
- Fig. 10: illustrates schematically an associated data logger (transmitter) as seen from the front,
- Fig. 11: shows the data logger (transmitter) of Fig. 10 as seen from the side,
- Fig. 12: shows the data logger (transmitter) of Fig. 10 as seen from the rear, and
- Fig. 13: illustrates schematically a perspective view of a complete functioning unit according to an embodiment of the invention with a nappy, a sensor and a data logger.

The figures are reproduced as examples of how the present invention can be implemented and should be regarded as non-limiting to the scope of protection as laid down in the claims.

### Detailed description of the invention

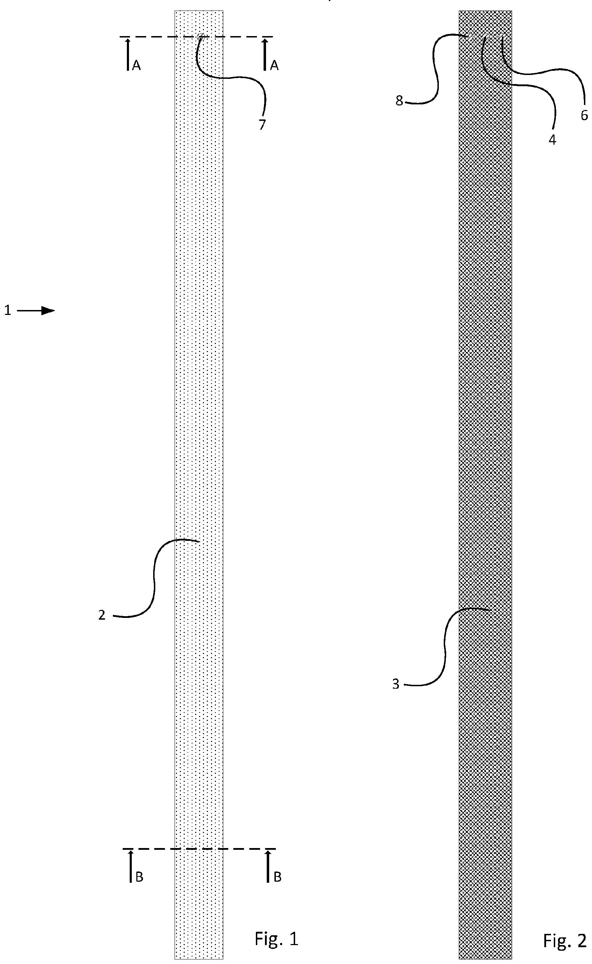

Fig. 1 shows an example of a complete sensor 1 according to an embodiment of the invention with a skin-friendly and moisture-proof electrically insulating outer layer 2, wherein the opening indicated through the outer layer 2 and a first non-liquid absorbing insulating layer (dielectric) 9 (not shown in Fig. 1) provides electrical access from the outside to a first electrode 7 for capacitive liquid detection. The outer layer 2 can, for example, consist of a plastic film.

Fig. 2 shows the same sensor 1 from the rear side, which is covered by a rear side layer 3, wherein an opening through the rear side layer 3 provides electrical access to a second and innermost electrode 4 for capacitive liquid sensing, and wherein two other openings through the rear side layer 3 and a second non-moisture absorbing insulating layer (dielectric) 5 (not shown in Fig. 2) provide electrical access to a first 6 and a second 8 electrode for capacitive faeces detection. The rear side layer 3 of sensor 1 is preferably an adhesive moisture-proof and electrically insulating layer, such as for example double-sided tape.

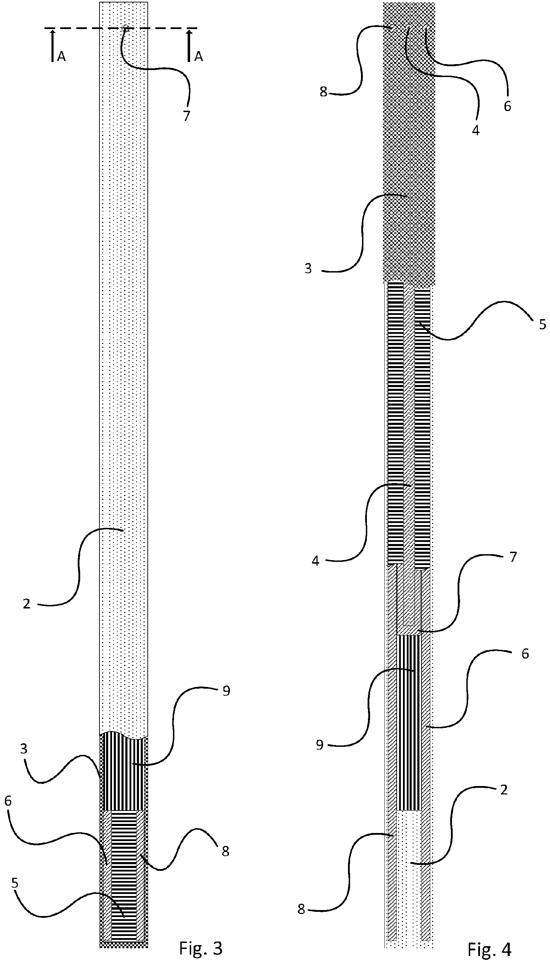

Fig. 3 shows the sensor 1 with the outer layer 2 shortened, thus exposing parts of the dielectrics 5, 9, the electrodes 6, 8 for capacitive faeces detection and the rear side layer 3. In fact, the outer layer 2 covers the entire sensor 1, but the inner layers 5, 6, 8,9 are shown in this way for explanatory reasons. The electrodes 6, 8 for capacitive faeces detection together constitute a capacitive sensor for detection of faeces.

Fig. 4 shows the sensor 1 from the rear side, this time with the rear side layer 3 shortened, thus exposing parts of second electrode 4 for capacitive liquid detection and of the second dielectric 5. In fact, the rear side layer 3 covers the entire sensor 1, but the inner layers 4-9 are shown in this way for explanatory reasons. The second dielectric 5 has also been shortened. In fact, it covers the entire sensor 1 as indicated in Fig. 3. The electrodes 4, 7 for capacitive liquid detection and the second dielectric 5 together constitute a capacitive sensor for detection of the quantity of liquid. The lengths of the electrodes 4, 7 for liquid detection correspond to the length of the liquid absorbing material for which measurements are desired. In Fig. 4, the electrodes 6, 8 for capacitive faeces detection, the first dielectric 9 and the outer layer 2 are also shown. The lengths of the electrodes 6, 8 for capacitive faeces detection are adjusted to fit the area in the nappy, in which faeces can be expected to occur.

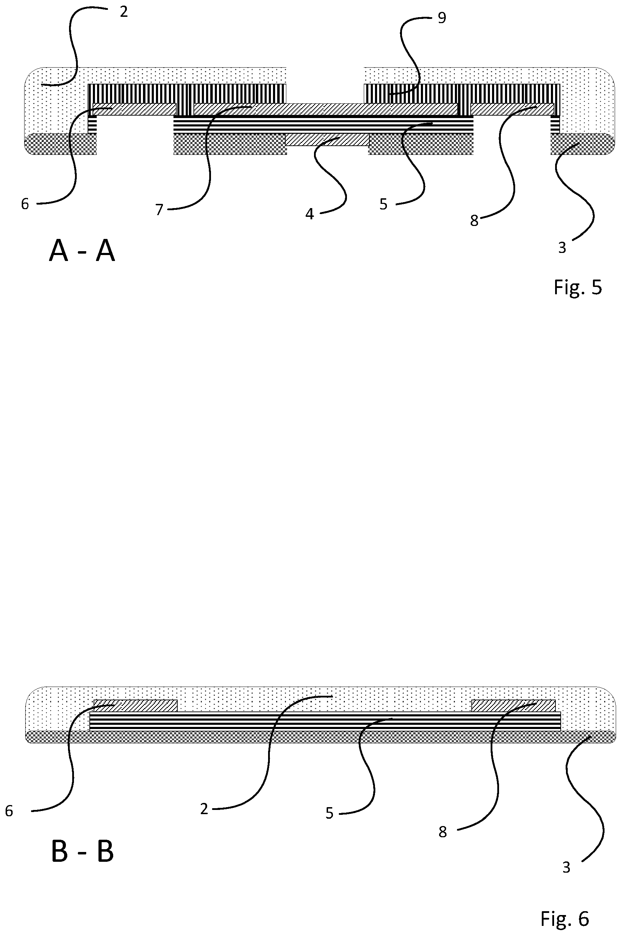

Fig. 5 shows a cross-section of the sensor 1 as indicated by the A-A line in Figs. 1 and 3. It illustrates schematically an example of a possible design of sensor 1 comprising an outer layer 2 and a rear side layer 3 as described above. The dielectrics 5, 9 consist of electrical insulating materials, such as for example foam tape, polyethylene foam, paper or an elastic liquid polymer applied in a printing process, which polymer subsequently solidifies. The four electrodes 4, 6, 7, 8 are made from electrically conductive materials, such as copper, aluminium, iron, graphite coating or a conductive polymer, and can be installed as tapes with an adhesive or applied in a printing process directly on the carriers constituted by the dielectrics 5 and 9.

Fig. 6 shows a cross-section of the sensor 1 as indicated by the B-B line in Fig. 1 with the outer layer 2 and the rear side layer 3 as described above. The electrodes 6, 8 for capacitive faeces detection as well as the second dielectric 5 are also shown as described previously.

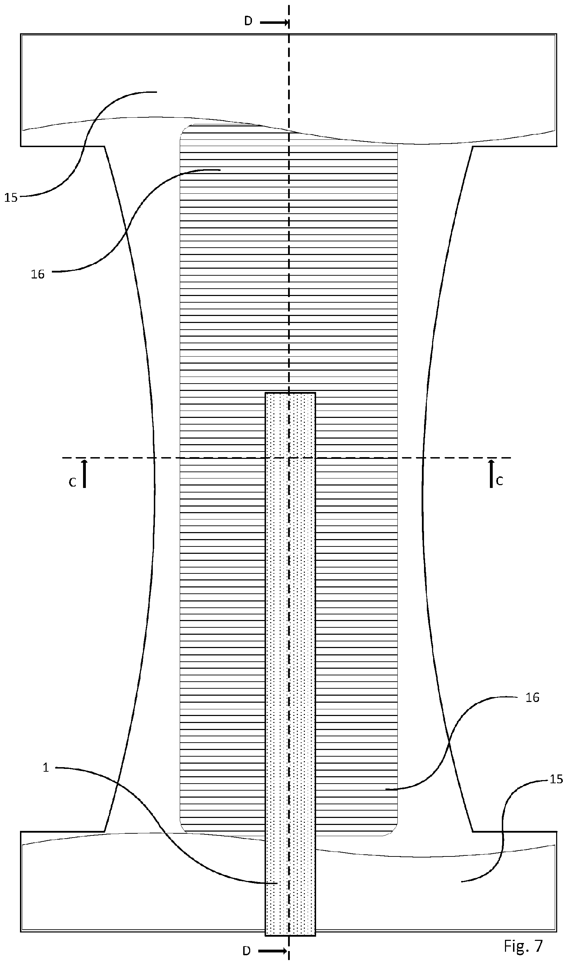

Fig. 7 shows an arbitrary type of nappy seen from the inside. The surface layer 15 of the nappy is shown partly open for explanatory reasons to illustrate the liquid absorbing material 16 inside the nappy. In fact, the surface layer 15 covers the entire nappy. Sensor 1 is seen from the rear edge of the nappy across the area within the nappy, in which faeces can be expected to occur. Thus, the sensor is arranged to measure the presence of faeces.

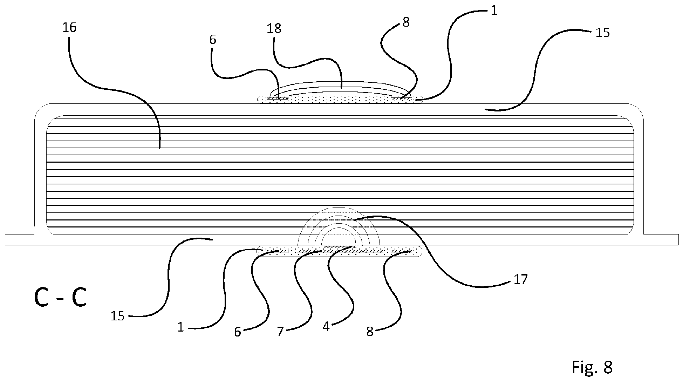

Fig. 8 shows a cross-section of the nappy and sensor 1 as indicated by the C-C line in Fig. 7. When an alternating voltage is applied across the electrodes 4, 7 of the capacitive liquid sensor, an electrical field 17 is formed within the liquid absorbing material 16. When an alternating voltage is applied across the electrodes 6, 8 of the capacitive faeces sensor, an electrical field 18 is formed between the surface of the sensor and the body of a person wearing the nappy across the area of the nappy, in which faeces can be expected to occur.

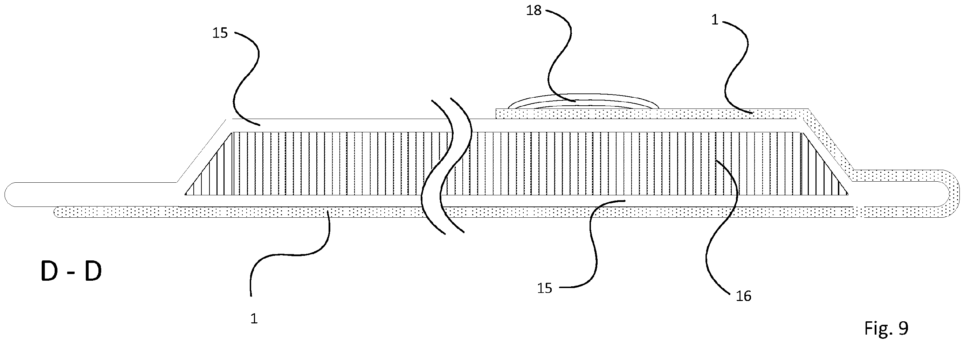

Fig. 9 shows a cross-section of the nappy and sensor 1 as indicated by the D-D line in Fig. 7. The sensor 1 may be fastened to the surface of the nappy, using, for example, glue. The electrical field 18, which is formed when an alternating voltage is applied across the electrodes 6, 8 of the capacitive faeces sensor, is also seen.

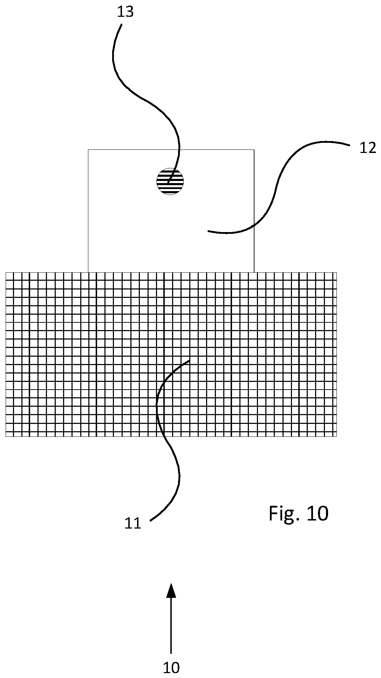

Fig. 10 is a schematic front view of a data logger 10 according to an embodiment of the invention, wherein a housing 11 contains the electronics (measuring circuit, accelerometer, GPS, GSM modem, power supply and radio module, etc.) and a battery, a rubber hinge 12 is installed for fastening the housing 11 across the front edge of the nappy by means of, for example, a magnet contact, and a body sensor 13 creates an electrical connection to the body of a person wearing the nappy. The data logger 10, the nappy and the sensor 1 together constitute a complete functional liquid and faeces detection unit 19.

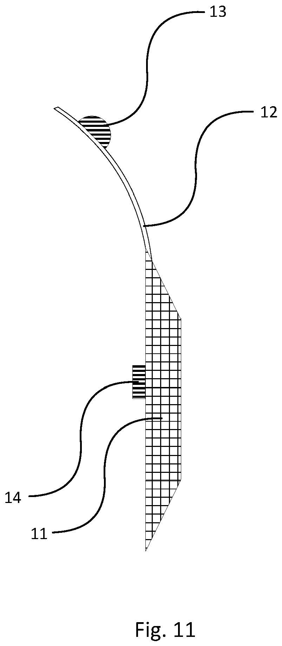

Fig. 11 shows the data logger 10 of Fig. 10 as seen from the side with the housing 11, the rubber hinge 12, the body sensor 13, and a plug 14 which creates electrical connections to the four electrodes 4, 6, 7, 8 of sensor 1.

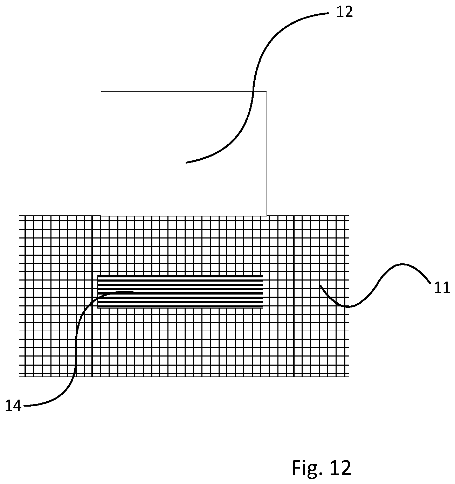

Fig. 12 is a rear view of the same data logger 10, wherein the plug 14 functions as a contact part for the four electrodes 4, 6, 7 and 8 of sensor 1. Thus, the plug 14 creates the electrical connection to the four electrodes 4, 6, 7, 8 through the openings designed for this purpose at the top of sensor 1 as seen in Figs. 1 and 2, such that the data logger 10 can easily be installed together with sensor 1.

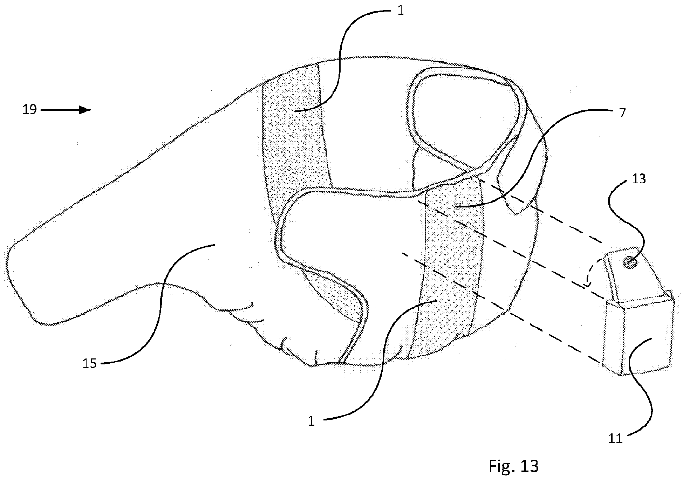

Fig. 13 shows an arbitrary nappy, onto the outside of which is fitted a sensor 1, and data logger 10, viewed in a perspective. The sensor 1 is centred in the nappy's longitudinal direction. These three components together constitute a liquid and faeces detection unit 19 functioning separately in the complete system.

The unit 19 functions by the data logger 10 applying an alternating voltage of, for example, 2 Vac at a frequency of, for example, 10 kHz across the two electrodes 4, 7 for capacitive liquid detection, concurrently measuring the capacitance between the electrodes 4, 7. Because the liquid sensor (consisting of the two electrodes 4, 7 and the second dielectric 5) creates an electrical field 17 into the liquid absorbing material 16 in the nappy, the capacitance measured will depend on the dielectric properties of the liquid absorbing material 16. This utilises the physical differences between a dry and a damp/wet liquid absorbing material 16. The dielectric constant for water (and urine) is approximately 80 times higher than the dielectric constant for air. The effect on the electrical field 17 of this difference in dielectric constants is utilised in this design. The relationship between the capacitance measured and the quantity of liquid in the nappy is approximately linear, always with a positive slope (the more liquid present, the higher the capacitance measured). Advantageously, the width of the second electrode 4 can be approximately 50 % of the width of the first electrode 7 as indicated roughly in Fig. 5.

The electrodes 6, 8 for capacitive faeces detection detect the presence of faeces in the nappy. The sensor functions by the data logger 10 applying an alternating voltage of, for example, 2 Vac at a frequency of, for example, 10 kHz between the two electrodes 6, 8 for capacitive faeces detection, concurrently measuring the capacitance between the electrodes 6, 8. These electrodes 6, 8 are electrically and capacitively insulated from the surroundings by means of the two dielectric layers 5, 9 as indicated in Figs. 3-5. As indicated in Figs. 3 and 4, the first dielectric 9 is shorter than the two electrodes 4, 7 and the second dielectric 5, which creates an opening to the electrodes 6, 8 at one end thereof. This opening provides increased capacitive sensitivity in the area, in which faeces are expected to occur. Thus, the capacitive faeces sensor consisting of the two electrodes 6, 8 and the two dielectrics 5, 9 forms an electrical field 18 with increased sensitivity in the area, in which the first dielectric 9 is not present. The dielectric constant for faeces is many times higher than the dielectric constant for air.

The effect on the electrical field 18 of this difference in dielectric constants is utilised in this design.

In a variant of the invention, the capacitive faeces sensor consists of the two electrodes 6, 8 and the two dielectrics 5, 9 as described above in combination with a body sensor 13. In this embodiment, the data logger 10 applies an alternating voltage of, for example, 2 Vac at a frequency of, for example, 10 kHz between the body sensor 13 on the one side and the two electrodes 6, 8 on the other side, concurrently measuring the capacitance between the body sensor 13 on the one side and the electrodes 6, 8 on the other side. The alternating voltage applied between the body sensor 13 and the two electrodes 6, 8 forms an electrical field between the body of a wearer of the complete unit 19 and the designed sensitive area with electrodes 6, 8, in which faeces are expected to occur. The electrical field and, consequently, the capacitance measured between the body and the electrodes 6, 8, is depend on the presence of faeces, because the dielectric constant for faeces is many times higher than the dielectric constant for air. The effect of this difference on the electrical field between the body and the electrodes 6, 8 is utilised in this design.

Increased reliability of diagnosing the presence of faeces in, for example, a nappy is obtained through a combination of measurements between the electrodes 6, 8 and the body via the body sensor 13.

Advantageously, the application of alternating voltages to the electrodes 4, 6, 7, 8 and the corresponding measurements of capacitance can be done at predefined time intervals of, for example, 10 seconds or 1 minute and temporally shifted in relation to each other in the sense that not more than one measurement is taken at any given time.

The capacitances measured between the two electrodes 4, 7 for capacitive liquid detection are equated to estimates of the state of fullness of liquid, whereas the capacitances measured between the two electrodes 6, 8 for capacitive faeces detection (and in some embodiments) the body of a wearer of a nappy, is equated to estimates of whether or not faeces are present in the nappy.

### List of reference numbers

1. Complete retrofitted sensor
2. Moisture-proof and electrically insulating outer layer of the sensor
3. Adhesive, moisture-proof and electrically insulating rear side layer of the sensor
4. Second and innermost electrode for capacitive liquid detection
5. Second non-moisture absorbing insulating layer (dielectric)
6. First electrode for capacitive faeces detection
7. First electrode for capacitive liquid detection
8. Second electrode for capacitive faeces detection
9. First non-liquid absorbing insulating layer (dielectric)
10. Complete data logger (transmitter) for fitting on sensor and nappy
11. Housing for data logger containing printed circuit board, accelerometer, GPS, GSM modem radio and battery, etc.
12. Rubber hinge for fastening the data logger to the nappy and for the body sensor
13. Body sensor for creating electrical connection to the wearer's body
14. Plug for electrical contact with the four electrodes in the sensor
15. Surface layer of an arbitrary standard nappy
16. Liquid-absorbing material within an arbitrary nappy
17. Electrical field from the capacitive liquid sensor
18. Electrical field from the capacitive faeces sensor
19. Assembled functional unit comprising nappy, sensor and data logger

## Claims

1. A system for measuring and recording a quantity of liquid in an absorbing material (16), which system comprises a sensor (1) comprising
a first pair of electrodes (4, 7) for measuring the quantity of liquid, which first pair of electrodes (4, 7) is arranged to be placed around the absorbing material (16), and
a data logger (10), which is arranged to apply an alternating voltage across the first pair of electrodes (4, 7) and, concurrently herewith, to measure the capacitance between the two electrodes of the first pair of electrodes (4, 7) and to calculate, based on the measured capacitance, an estimate of the quantity of liquid in the absorbing material (16), **characterised in that**
the sensor (1) with the one pair of electrodes (4, 7) is arranged to be placed so that it extends along two opposite surfaces of the absorbing material (16).

2. A system according to claim 1, **characterised in that** the two opposing surfaces are two surfaces of a nappy facing towards the wearer and away from the wearer, respectively.

3. The system according to claim 1 or 2, wherein the sensor further comprises a second pair of electrodes (6, 8), and wherein the data logger (10) is further arranged to be able to apply an alternating voltage across the second pair of electrodes (6, 8) and, concurrently herewith, to measure the capacitance between the two electrodes of the second pair of electrodes (6, 8) and to estimate, based on the measured capacitance, whether a substance, for example faeces, is present adjacent the two electrodes of the second pair of electrodes (6, 8).

4. The system according to claim 3, **characterised in that** the data logger (10) further comprises a contact surface (13), which is arranged to be placed in electrical contact with a body of a person, and wherein the data logger (10) is further arranged to be able to apply an alternating voltage between the two electrodes of the second pair of electrodes (6, 8) on the one side and this contact surface on the other side and, concurrently herewith, measure the capacitance between the two electrodes of the second pair of electrodes (6, 8) on the one side and the contact surface on the other side and to estimate, based on the measured capacitance, whether a substance, for example faeces, is present between the two electrodes of the second pair of electrodes (6, 8) and the body.

5. The system according to claim 4, **characterised in that** a temperature-measuring instrument is arranged in connection with the contact surface of the data logger (10).

6. The system according to any one of the preceding claims, **characterised in that** the data logger (10) is provided with a gyroscope and/or an accelerometer.

7. The system according to any one of the preceding claims, **characterised in that** the data logger (10) is provided with a GPS tracking module for localising the data logger (10).

8. The system according to any one of the preceding claims, **characterised in that** the data logger (10) is provided with a GSM modem for telecommunication with the data logger (10).

9. The system according to any one of the preceding claims, **characterised in that** the alternating voltage applied has an amplitude of between 0.5 Vac and 15 Vac, preferably between 0.5 Vac and 10 Vac, most preferably between 1 Vac and 6 Vac.

10. The system according to any one of the preceding claims, **characterised in that** the alternating voltage applied has a frequency of between 1 kHz and 100 kHz, preferably between 5 kHz and 25 kHz, most preferably between 8 kHz and 12 kHz.

11. The system according to any one of the preceding claims, **characterised in that** the alternating voltage is applied for short periods at predefined intervals of between 5 seconds and 10 minutes, preferably between 5 seconds and 1 minute.

12. The system according to any one of the preceding claims, **characterised in that** the data logger is further arranged to be able to transmit signals, which are representative of the calculated estimations of a quantity of liquid or of the presence of a substance, wirelessly to one or more external monitoring stations.

13. The system according to claim 12, **characterised in that** the signals, which are representative of a quantity of liquid, represent an absolute quantity of liquid, such as for example 300 ml.

14. The system according to claim 12, **characterised in that** the signals, which are representative of a quantity of liquid, represent a relative quantity of liquid, such as for example 20% of the absorbing material's (16) total capacity.

## Patentansprüche

1. System zum Messen und Aufzeichnen einer Menge an Flüssigkeit in einem absorbierenden Material (16), wobei das System einen Sensor (1) umfasst, umfassend:
ein erstes Paar Elektroden (4, 7) zum Messen der Menge an Flüssigkeit, wobei das erste Paar von Elektroden (4, 7) um das absorbierende Material (16) herum platziert werden soll, und
einen Datenlogger (10), der eine Wechselspannung über dem ersten Paar von Elektroden (4, 7) anlegen und gleichzeitig damit die Kapazität zwischen den beiden Elektroden des ersten Paares von Elektroden (4, 7) messen und basierend auf der gemessenen Kapazität eine Schätzung der Menge an Flüssigkeit in dem absorbierenden Material (16) berechnen soll, **dadurch gekennzeichnet, dass**
der Sensor (1) mit dem einen Paar von Elektroden (4, 7) so zu platzieren ist, dass er sich entlang zweier entgegengesetzter Oberflächen des absorbierenden Materials (16) erstreckt.

2. System nach Anspruch 1, **dadurch gekennzeichnet, dass** die beiden entgegengesetzten Oberflächen zwei Oberflächen einer Windel sind, die zu dem Träger beziehungsweise von dem Träger weg weisen.

3. System nach Anspruch 1 oder 2, wobei der Sensor des Weiteren ein zweites Paar von Elektroden (6, 8) umfasst, und wobei der Datenlogger (10) des Weiteren in der Lage sein soll, eine Wechselspannung über dem zweiten Paar von Elektroden (6, 8) anzulegen und gleichzeitig damit die Kapazität zwischen den beiden Elektroden des zweiten Paares von Elektroden (6, 8) zu messen und basierend auf der gemessenen Kapazität zu schätzen, ob eine Substanz, beispielsweise Fäkalien, benachbart zu den beiden Elektroden des zweiten Paares von Elektroden (6, 8) vorhanden ist.

4. System nach Anspruch 3, **dadurch gekennzeichnet, dass** der Datenlogger (10) des Weiteren eine Kontaktoberfläche (13) umfasst, die in elektrischem Kontakt mit einem Körper einer Person zu platzieren ist, und wobei der Datenlogger (10) des Weiteren in der Lage sein soll, eine Wechselspannung zwischen den beiden Elektroden des zweiten Paares von Elektroden (6, 8) auf der einen Seite und dieser Kontaktoberfläche auf der anderen Seite anzulegen und gleichzeitig damit die Kapazität zwischen den beiden Elektroden des zweiten Paares von Elektroden (6, 8) auf der einen Seite und der Kontaktoberfläche auf der anderen Seite zu messen, und um basierend auf der gemessenen Kapazität abzuschätzen, ob eine Substanz, beispielsweise Fäkalien, zwischen den beiden Elektroden des zweiten Paares von Elektroden (6, 8) und dem Körper vorhanden ist.

5. System nach Anspruch 4, **dadurch gekennzeichnet, dass** ein Temperaturmessinstrument in Verbindung mit der Kontaktoberfläche des Datenloggers (10) angeordnet ist.

6. System nach einem der vorhergehenden Ansprüche, **dadurch gekennzeichnet, dass** der Datenlogger (10) mit einem Gyroskop und/oder einem Beschleunigungsmesser ausgestattet ist.

7. System nach einem der vorhergehenden Ansprüche, **dadurch gekennzeichnet, dass** der Datenlogger (10) mit einem GPS-Trackingmodul ausgestattet ist, um den Datenlogger (10) zu lokalisieren.

8. System nach einem der vorhergehenden Ansprüche, **dadurch gekennzeichnet, dass** der Datenlogger (10) mit einem GSM-Modem zur Telekommunikation mit dem Datenlogger (10) ausgestattet ist.

9. System nach einem der vorhergehenden Ansprüche, **dadurch gekennzeichnet, dass** die angelegte Wechselspannung eine Amplitude zwischen 0,5 V AC und 15 V AC, vorzugsweise zwischen 0,5 V AC und 10 V AC, am meisten bevorzugt zwischen 1 V AC und 6 V AC hat.

10. System nach einem der vorhergehenden Ansprüche, **dadurch gekennzeichnet, dass** die angelegte Wechselspannung eine Frequenz zwischen 1 kHz und 100 kHz, vorzugsweise zwischen 5 kHz und 25 kHz, am meisten bevorzugt zwischen 8 kHz und 12 kHz hat.

11. System nach einem der vorhergehenden Ansprüche, **dadurch gekennzeichnet, dass** die Wechselspannung für kurze Perioden in vordefinierten Intervallen zwischen 5 Sekunden und 10 Minuten, vorzugsweise zwischen 5 Sekunden und 1 Minute angelegt wird.

12. System nach einem der vorhergehenden Ansprüche, **dadurch gekennzeichnet, dass** der Datenlogger des Weiteren in der Lage sein soll, Signale, die für die berechneten Schätzungen einer Menge an Flüssigkeit oder das Vorhandensein einer Substanz repräsentativ sind, drahtlos an eine oder mehrere externe Überwachungsstationen zu übertragen.

13. System nach Anspruch 12, **dadurch gekennzeichnet, dass** die Signale, die für eine Menge an Flüssigkeit repräsentativ sind, eine absolute Menge an Flüssigkeit repräsentieren, wie beispielsweise 300 ml.

14. System nach Anspruch 12, **dadurch gekennzeichnet, dass** die Signale, die repräsentativ für eine Menge an Flüssigkeit sind, eine relative Menge an Flüssigkeit repräsentieren, wie beispielsweise 20 % der Gesamtkapazität des absorbierenden Materials (16).

## Revendications

1. Système destiné à mesurer et enregistrer une quantité de liquide dans un matériau absorbant (16), lequel système comprend un capteur (1) comprenant
une première paire d'électrodes (4, 7) pour mesurer la quantité de liquide, laquelle première paire d'électrodes (4, 7) est agencée pour être placée autour du matériau absorbant (16), et
un enregistreur de données (10), qui est agencé pour appliquer une tension alternative à travers la première paire d'électrodes (4, 7) et, simultanément à cela, pour mesurer la capacité entre les deux électrodes de la première paire d'électrodes (4, 7) et pour calculer, sur la base de la capacité mesurée, une estimation de la quantité de liquide dans le matériau absorbant (16), **caractérisé en ce que**
le capteur (1) avec la première paire d'électrodes (4, 7) est agencé pour être placé de telle sorte qu'il s'étend le long de deux surfaces opposées du matériau absorbant (16).

2. Système selon la revendication 1, **caractérisé en ce que** les deux surfaces opposées sont deux surfaces d'une couche-culotte faisant face au porteur et tournant le dos au porteur, respectivement.

3. Système selon la revendication 1 ou 2, dans lequel le capteur comprend en outre une deuxième paire d'électrodes (6, 8), et dans lequel l'enregistreur de données (10) est en outre agencé pour pouvoir appliquer une tension alternative à travers la deuxième paire d'électrodes (6, 8) et, simultanément à cela, pour mesurer la capacité entre les deux électrodes de la deuxième paire d'électrodes (6, 8) et pour estimer, sur la base de la capacité mesurée, si une substance, par exemple des selles, est présente au voisinage des deux électrodes de la deuxième paire d'électrodes (6, 8).

4. Système selon la revendication 3, **caractérisé en ce que** l'enregistreur de données (10) comprend en outre une surface de contact (13), qui est agencée pour être placée en contact électrique avec un corps d'une personne, et dans lequel l'enregistreur de données (10) est en outre agencé pour pouvoir appliquer une tension alternative entre les deux électrodes de la deuxième paire d'électrodes (6, 8) sur le premier côté et cette surface de contact sur l'autre côté et, simultanément à cela, mesurer la capacité entre les deux électrodes de la deuxième paire d'électrodes (6, 8) sur le premier côté et la surface de contact sur l'autre côté et pour estimer, sur la base de la capacité mesurée, si une substance, par exemple des selles, est présente entre les deux électrodes de la deuxième paire d'électrodes (6, 8) et le corps.

5. Système selon la revendication 4, **caractérisé en ce qu'**un instrument de mesure de température est agencé en connexion avec la surface de contact de l'enregistreur de données (10).

6. Système selon l'une quelconque des revendications précédentes, **caractérisé en ce que** l'enregistreur de données (10) est pourvu d'un gyroscope et/ou d'un accéléromètre.

7. Système selon l'une quelconque des revendications précédentes, **caractérisé en ce que** l'enregistreur de données (10) est pourvu d'un module de suivi GPS pour localiser l'enregistreur de données (10).

8. Système selon l'une quelconque des revendications précédentes, **caractérisé en ce que** l'enregistreur de données (10) est pourvu d'un modem GPS pour des télécommunications avec l'enregistreur de données (10).

9. Système selon l'une quelconque des revendications précédentes, **caractérisé en ce que** la tension alternative appliquée a une amplitude comprise entre 0,5 Vac et 15 Vac, de préférence entre 0,5 Vac et 10 Vac, idéalement entre 1 Vac et 6 Vac.

10. Système selon l'une quelconque des revendications précédentes, **caractérisé en ce que** la tension alternative appliquée a une fréquence comprise entre 1 kHz et 100 kHz, de préférence entre 5 kHz et 25 kHz, idéalement entre 8 kHz et 12 kHz.

11. Système selon l'une quelconque des revendications précédentes, **caractérisé en ce que** la tension alternative est appliquée pendant de courtes périodes à intervalles prédéfinis compris entre 5 secondes et 10 minutes, de préférence entre 5 secondes et 1 minute.

12. Système selon l'une quelconque des revendications précédentes, **caractérisé en ce que** l'enregistreur de données est en outre agencé pour pouvoir transmettre des signaux, qui sont représentatifs des estimations calculées d'une quantité de liquide ou de la présence d'une substance, sans fil à une ou plusieurs stations de surveillance externes.

13. Système selon la revendication 12, **caractérisé en ce que** les signaux, qui sont représentatifs d'une quantité de liquide, représentent une quantité absolue de liquide, comme par exemple 300 ml.

14. Système selon la revendication 12, **caractérisé en ce que** les signaux, qui sont représentatifs d'une quantité de liquide, représentent une quantité relative de liquide, comme par exemple 20 % de la capacité totale du matériau absorbant (16).
